# EUROPÄISCHE PATENTSCHRIFT

(11) **EP 1 971 297 B1**
(45) Veröffentlichungstag und Bekanntmachung des Hinweises auf die Patenterteilung: **07.03.2012**
(21) Anmeldenummer: 06828627.7
(22) Anmeldetag: 07.12.2006
(51) Int. Cl.: A61F 2/58, A61F 2/68

(54) **HANDPROTHESE MIT GELENKIG AUSRICHTBAREN FINGERN**
HAND PROSTHESIS
PROTHESE DE MAIN

(30) Priorität: 20.12.2005 DE 102005061313
(43) Veröffentlichungstag der Anmeldung: 24.09.2008
(73) Patentinhaber: Otto Bock HealthCare GmbH, 37115 Duderstadt (DE)
(72) Erfinder: PUCHHAMMER, Gregor, A-1130 Wien (AT)
(74) Vertreter: Stornebel, Kai
(86) Internationale Anmeldenummer: PCT/DE2006/002177
(87) Internationale Veröffentlichungsnummer: WO 2007/076765

(56) Entgegenhaltungen:
- EP-A1- 1 195 151
- DE-U1- 20 301 116
- GB-A- 1 585 256
- US-A- 2 553 827
- US-A- 4 364 593
- US-A- 4 623 354
- US-A1- 2003 195 638

## Beschreibung

Die Erfindung betrifft eine Handprothese mit einem Chassis, an dem mehrere Fingerprothesen gelenkig gelagert sind, die über einen Antrieb um zumindest eine Schwenkachse relativ zu dem Chassis und aufeinander zu bewegbar sind.

Die Aufgabe einer Handprothese ist es, die Optik und die Funktion einer Hand, die ersetzt werden musste, möglichst naturgetreu zu ersetzen. Dazu muss die Handprothese in der Lage sein, Greifeinrichtungen, die als Fingernachbildungen ausgebildet sein können, zueinander zu verlagern, um ein Greifen eines Gegenstandes zu ermöglichen.

Aus der US 2004/00195638 A1 ist ein Zwei-Finger-Greifer bekannt, bei dem zwei Greifeinrichtungen aus einer geöffneten Stellung in eine geschlossene Stellung verlagert werden können, in der die Greifeinrichtungen unmittelbar einander gegenüberliegen. Ein zwischen den Greifeinrichtungen befindlicher Gegenstand kann so gehalten werden. Zum Lösen des Griffes kann eine Drehrichtungsumkehr des Antriebes eingeleitet werden.

Aus der WO 03/017880 A1 ist eine Prothesenhand bekannt, bei der in jeder einzelnen Fingerprothese, die an einem Chassis gelagert ist, ein separater Antrieb angeordnet ist. Mit einer solchen Prothesenhand ist es möglich, verschiedene Griffsituationen zu realisieren, beispielsweise einen Spitzgriff oder einen Lateralgriff. Nachteilig ist der hohe Steuerungsaufwand für jeden einzelnen Finger, die aufwendige Technik mit in den Finger integrierten Antrieben sowie eine erhöhte Störanfälligkeit aufgrund der komplexen Bauweise.

Die US 2003/195638 A1 beschreibt eine Greifeinrichtung mit einem Motor, der über ein Spindelgetriebe und eine Hebelkonstruktion angetrieben ist. Über ein Spindelgetriebe wird bei einer Umkehrung der Drehrichtung ein Öffnen oder ein Schließen der Greifelemente bewirkt.

Die DE 405 871 B1 beschreibt eine künstliche Hand, die aus einem Handteller und an diesem drehbaren Fingern und Daumen besteht. Im Handteller ist eine um eine zur Handfläche senkrechte Achse drehbare Antriebsscheibe durch Verbindungen an die Fingerglieder derart angeschlossen, dass die Drehung der Scheibe in eine Richtung das Öffnen und in entgegengesetzter Richtung das Schließen der Finger bewirkt. Die Drehung der Scheibe in die eine Richtung wird durch eine Schnur bewirkt, die Bewegung in entgegengesetzter Richtung wird durch eine Feder im Inneren der Scheibe bewirkt. Am Umfang der Antriebsscheibe sind Sperrzähne angebracht, die mit einer Sperrklinke in Eingriff kommen und die Antriebsscheibe in derjenigen Stellung festhalten, welche sie in ihrer Drehung erreicht hat.

Ausgehend von diesem Stand der Technik liegt der Erfindung die Aufgabe zugrunde, eine Handprothese bereitzustellen, die eine einfache Steuerung aufweist, zuverlässig arbeitet und preiswert herzustellen ist.

Erfindungsgemäß wird diese Aufgabe durch eine Handprothese mit den Merkmalen des Anspruchs 1 gelöst. Vorteilhafte Ausgestaltungen und Weiterbildungen der Erfindung sind in den Unteransprüchen aufgeführt.

Die erfindungsgemäße Handprothese mit einem Chassis, an dem mehrere Fingerprothesen gelenkig gelagert sind, die über einen Antrieb um zumindest eine Schwenkachse relativ zu dem Chassis und aufeinander zu bewegbar sind, sieht vor, dass die Kraftübertragungseinrichtungen an einem gemeinsamen Antrieb dergestalt mit den Fingerprothesen gekoppelt sind, dass ausgehend von einer Ruhestellung der Fingerprothesen in Abhängigkeit von der Drehrichtung des Antriebes zumindest zwei Fingerprothesen unterschiedliche Verstellwinkel relativ zu dem Chassis durchlaufen. Wird der Antrieb in die eine Drehrichtung aktiviert, bewegen sich aus einer Ruhestellung beispielsweise zunächst der Zeige- und Mittelfinger in Richtung Handinnenfläche, während der Daumen später oder langsamer aktiviert wird. Dann kann mit diesen drei Fingern der sogenannte "Lateralgriff" realisiert werden. Bei der anderen Drehrichtung, ausgehend von der Ruhestellung, in der die Handprothese eine geöffnete Haltung innehat, wird zunächst der Daumen aktiviert oder schneller in Richtung Handinnenfläche bewegt, so dass die Spitzen der Fingerprothesen zusammengeführt werden, um einen "Spitzgriff" zu realisieren. Es findet also eine unterschiedliche zeitliche Abfolge der Bewegung in Abhängigkeit von der Drehrichtung des Antriebes statt, wobei die Fingerprothesen mechanisch mit dem Antrieb gekoppelt sind, so dass mit einem geringen Steuerungsaufwand, nämlich mit einer simplen Drehrichtungsumkehr, zwei verschiedene Griffzustände eingestellt werden können, mit denen die häufigsten Greifverrichtungen ausgeführt werden können. Eine besonders einfache Realisierung der unterschiedlichen Verstellwinkel wird erreicht, indem die KraftÜbertragungseinrichtungen dergestalt mit dem Kopplungselement gekoppelt sind, dass ihre antriebsseitigen Lagerungen unterschiedliche Totpunktlagen aufweisen. Bei einer Lagerung einer Kraftübertragungseinrichtung an einer Drehscheibe werden im Verlauf der Drehbewegung der Drehscheibe Verlagerungen in einer Bewegungskomponente in Gestalt einer Sinuskurve realisiert. In Abhängigkeit von dem zurückgelegten Drehwinkel werden unterschiedliche Verlagerungen in einer Richtungskomponente bewirkt. Durch die drehbare Lagerung der Kraftübertragungseinrichtung an dem Kopplungselement wird nur die Verlagerung in einer Richtungskomponente wirksam. Sind nun die Lagerpunkte der Kraftübertragungseinrichtungen auf dem Kopplungselement oder der Drehscheibe so gewählt, dass bei Aktivierung in der ersten Drehrichtung zunächst der Daumen eine Totpunktlage durchläuft, werden zunächst die übrigen Prothesenfinger, beispielsweise der Zeige- und der Mittelfinger verlagert. während bei entgegengesetzter Drehrichtung der Verstellweg für den Zeige- und Mittelfinger kleiner als der für den Daumen ist. Alternativ kann die Realisierung unterschiedlicher Verstellwinkel über das Aufrollen einer Kraftübertragungseinrichtung auf eine Kurvenscheibe erfolgen, deren Radius für jede Drehrichtung unterschiedlich ist. Wird ausgehend von einer Ruhestellung die Kurvenscheibe in die erste Drehrichtung bewegt, werden die Kraftübertragungseinrichtungen, beispielsweise in Gestalt von Zugbänder, auf eine Kurvenscheibe mit einem größeren Radius als die Kraftübertragungseinrichtung für den Daumen aufgerollt. Dadurch wird zunächst der Zeige- und Mittelfinger palmar verlagert, während der Daumen nachfolgt. In entgegengesetzter Drehrichtung erfolgt dies entsprechend umgekehrt. Eine Rückstellbewegung kann über eine Federvorspannung der Fingerprothesen erfolgen. Durch die Kurvenscheiben können auch unterschiedliche Bewegungsabläufe der einzelnen Fingerprothesen eingestellt werden, beispielsweise dass zunächst eine hohe Verschwenkgeschwindigkeit bewirkt wird, die mit zunehmendem Verschwenkwinkel der Fingerprothesen abnimmt oder umgekehrt.

Neben den unterschiedlichen Verstellwinkeln, die entweder einen Spitzgriff oder einen Lateralgriff realisieren, können auch unterschiedliche Stellungen des Spitzgriffes bei entsprechender Abstimmung der Mechanik verwirklicht werden. Die mechanische Kopplung ist sehr preiswert herzustellen. Darüber hinaus wird nur ein einziger, gemeinsamer Antrieb benötigt, der bevorzugt in dem Chassis der Prothesenhand untergebracht ist, der aufgrund der im Vergleich zu den Fingerprothese großzügigen Platzverhältnisse wesentlich leistungsfähiger ausgelegt sein kann.

Eine Weiterbildung der Erfindung sieht vor, dass die Kraftübertragungseinrichtungen drehbar an einem verschwenkbaren Kopplungselement gelagert sind, beispielsweise einer Drehscheibe, um mit minimalen Nebenkräften, die aus Materialbiegungen bei einer starren Lagerung resultieren, die gewünschten Kräfte übertragen zu können. Das Kopplungselement selbst kann dreh- oder verschwenkbar ausgebildet sein, wobei eine besonders einfache Ausgestaltung darin besteht, dass eine Drehscheibe innerhalb des Chassis so angeordnet ist, dass die Drehachse des Kopplungselementes im Wesentlichen orthogonal zu der palmaren Oberfläche des Chassis verläuft. Zwischen dem Antrieb und dem Kopplungselement kann ein Getriebe angeordnet sein, um gegebenenfalls die erforderliche Untersetzung bereitzustellen, Bevorzugt liegt die Abtriebsachse des Antriebes ebenfalls orthogonal zu der palmaren Oberfläche des Chassis, so dass die gegebenenfalls erforderlichen Getriebestufen stets mit parallelen Drehachsen arbeiten können. Sollte aufgrund der Geometrie des Antriebes oder des Chassis eine Veränderung der Drehrichtung oder Orientierung der Drehachse notwendig sein, kann dies beispielsweise über ein Winkelgetriebe realisiert werden.

Um hohe Kräfte übertragen zu können, ist der Antrieb bevorzugt als ein Flachläufermotor ausgebildet, der einfach in dem Chassis, das in Gestalt der Metacarpus ausgebildet sein kann, untergebracht werden kann. Der bevorzugt als langsam laufender Motor ausgebildete Flachläufermotor kann bei relativ kompakter Bauweise und geringen Drehzahlen hohe Momente erzeugen. Die Drehzahlen können über ein Zykloiden-Getriebe oder ein Harmonic-Drive-Getriebe weiter auf die gewünschte Drehzahl reduziert werden.

Eine Weiterbildung der Erfindung sieht vor, dass die Kraftübertragungseinrichtungen zugstarr und drucknachgiebig oder biegeelastisch ausgebildet sind, so dass in einem begrenzten Maße eine Elastizität bei einer palmaren Kraftübertragung auf die Fingerprothesen möglich ist, während umgekehrt ein Öffnen der Prothesenhand ohne Entriegelung des Antriebes oder eine Drehrichtungsumkehr nicht möglich ist. Dadurch wird ein sicherer Griff gewährleistet. Die Kraftübertragungseinrichtungen können in einem gewissen Maße druckstabil sein, um Druckkräfte, gegebenenfalls zur Unterstützung einer Öffnungsbewegung, bereitstellen zu können.

Zur stabilen Übertragung von Zugkräften ist es vorgesehen, dass die Kraftübertragungskomponente eine Seil, - Litzen- oder Faserkomponente aufweist, wobei nachfolgend aus Gründen der Einfachheit nur von Seilkomponenten gesprochen wird. Die anderen Komponenten sind entsprechend darin eingeschlossen.

Die Seilkomponente kann als offene, geschlossene oder verdrehte Schlaufe ausgebildet sein und eine Elastomerkomponente aufweisen, um eine allseitige Verlagerung, beispielsweise bei Achsfehlstellungen, zu ermöglichen. Weiterhin schützt die Elastomerkomponente die Seilkomponente vor äußeren Einflüssen, wenn sie die Seilkomponente zumindest teilweise umschließt.

In der Kraftübertragungseinrichtung können Lagerbuchsen zur Aufnahme von Achsen angeordnet sein, die dem Chassis bzw. Antrieb und den Fingerprothesen zugeordnet sind. Bei einer federelastischen Ausgestaltung der Kraftübertragungseinrichtungen sind die Federraten bevorzugt so bemessen, dass bei einer Beaufschlagung der Kraftübertragungseinrichtung mit einer Druckkraft eine Rückstellung der Fingerprothese in eine Ausgangsstellung bewirkt wird.

Nachfolgend wird ein Ausführungsbeispiel der Erfindung anhand der Figuren näher erläutert. Es zeigen:
- Figur 1 -: eine schematische Darstellung einer Handprothese;
- Figur 2-: eine schematische Darstellung des Funktionsaufbaus einer Handprothese in palmarer Draufsicht;
- Figur 3 -: eine Seitenansicht der Figur 2,
- Figur 4 -: eine geschlossene Hand in Lateralgriff;
- Figur 5-: eine geschlossene Hand in Spitzgriff; sowie
- Figur 6 -: eine Einzeldarstellung einer Daumenprothese.

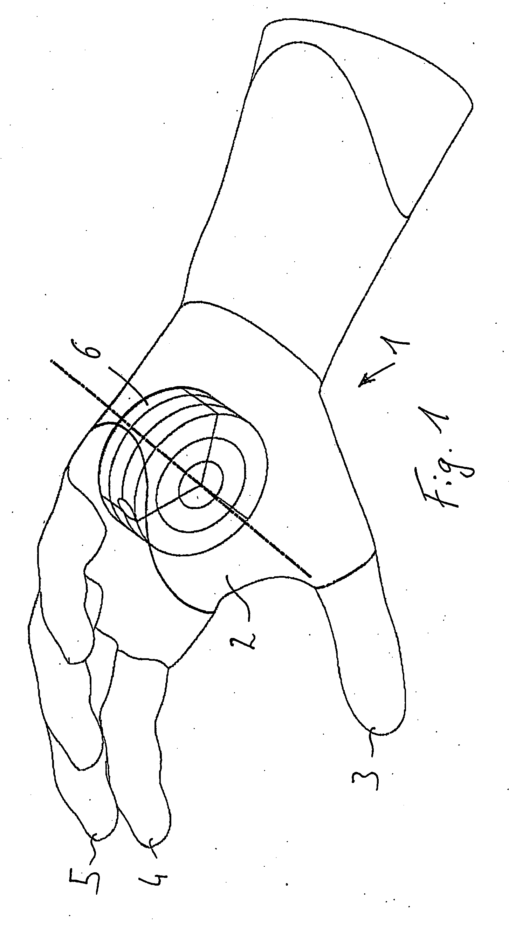

In der Figur 1 ist eine Handprothese 1, bestehend aus einem Handchassis 2 und zumindest drei gelenkig an dem Handchassis 2 gelagerten Fingerprothesen 3, 4, 5 gezeigt. Die Fingerprothesen 3, 4, 5 entsprechen dem Daumen, Zeigefinger und Mittelfinger einer natürlichen Hand. Eine bewegliche und über einen gemeinsamen Antrieb 6 aktuierbare Lagerung dieser drei Fingerprothesen 3, 4, 5 reicht aus, um die Mehrzahl der Greifverrichtungen einer Hand ausführen zu können. Die beiden übrigen Finger, der Ringfinger und der kleine Finger, können passiv mitbewegt werden und aus einem Elastomermaterial bestehen, um eine möglichst natürliche Anmutung zu erzielen. Innerhalb des Handchassis 2 ist der Antrieb 6 in Gestalt eines Elektromotors mit einem dazugehörigen Getriebe gelagert. Ebenfalls kann eine nicht dargestellte Energiequelle für den Antrieb 6 innerhalb des Handchassis 2 angeordnet sein. Eine Ansteuerung des Antriebs 6 erfolgt über ein Steuergerät, das in dem Handchassis 2 angeordnet sein kann. Die entsprechenden Signale können über eine Fernbedienung generiert oder als myoelektrische Signale ausgebildet sein.

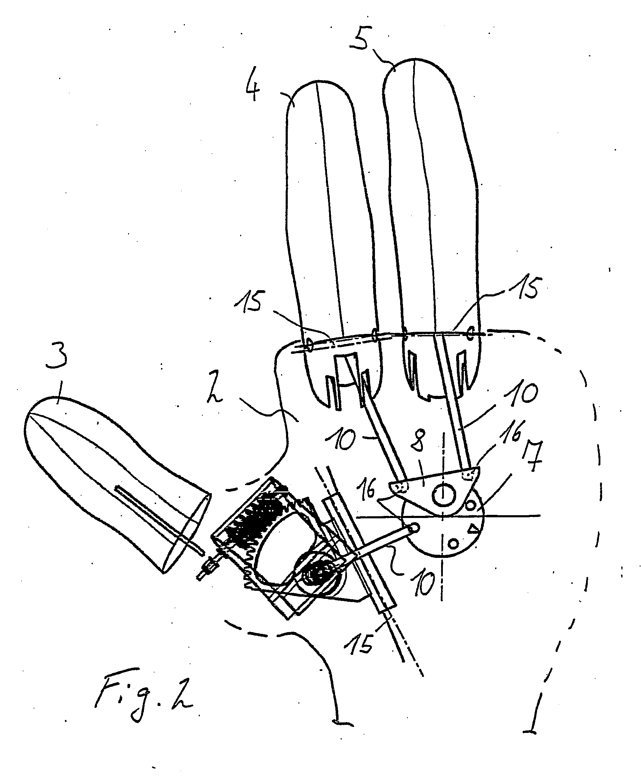

In der Figur 2 ist eine schematische Darstellung der Funktionsweise der Handprothese 1 gezeigt. An dem Handchassis 2 sind die drei Fingerprothesen 3, 4, 5 um Gelenkachsen 15 verschwenkbar gelagert. Die Fingerprothesen 3, 4, 5 sind über Kraftübertragungseinrichtungen 10, deren Aufbau weiter unten detailliert beschrieben werden wird, mit einer Drehscheibe 7 verbunden, die von dem Elektromotor 6 angetrieben wird. Die Kraftübertragungseinrichtungen 10 sind an der Drehscheibe 7 auf Achsen 16 entweder direkt oder über eine Wippe 8 gelagert. Der Zeigefinger 4 und der Ringfinger 5 sind über die Wippe 8, die drehbar auf der Drehscheibe 7 gelagert ist, miteinander gekoppelt. Die Drehscheibe 7 selbst ist entweder unmittelbar auf einer Abtriebswelle des Antriebes 6 oder einer Getriebeausgangswelle gelagert. Wird der Antrieb 6 aktiviert, wird die Drehscheibe 7 um einen entsprechenden Drehwinkel bewegt. Dadurch verlagern sich die Achsen 16 relativ zu den Schwenkachsen 15 der Fingerprothesen 3, 4, 5, was aufgrund der zugstarren Ausbildung der Kraftübertragungseinrichtungen 10 und einer von den Drehachsen 15 beabstandeten Anlenkung der Kraftübertragungseinrichtungen 10 an den Fingerprothesen 3, 4, 5 zu einer Verschwenkung der Fingerprothesen 3, 4, 5 führt. Wird der Antrieb 6 reversiert und bewegt sich die Drehscheibe 7 in eine Position, in der die Achsen 16 eine minimale Entfernung zu den Schwenkachsen 15 der Fingerprothesen 3, 4, 5 aufweisen, ist die geöffnete Ausgangsstellung oder Ruhestellung erreicht. Durch die federelastischen Eigenschaften der Kraftübertragungseinrichtungen 10 werden dann die Fingerprothesen 3, 4, 5 in ihre geöffnete Ausgangsstellung bewegt. Dabei ist es vorgesehen, dass die Kraftübertragungseinrichtungen 10 wesentlich höhere Zugkräfte als Druckkräfte übertragen können. Dies entspricht den physiologischen Gegebenheiten einer natürlichen Hand, die beim Schließen der Hand wesentlich größere Kräfte als beim Öffnen aufbringen kann.

Aus Gründen der Übersichtlichkeit sind der Ringfinger und der kleine Finger nicht dargestellt, diese können passiv an dem Mittelfinger 5 angelenkt und dadurch mitbewegt werden. Auch können der Ringfinger und der kleine Finger an der erweiterten Wippe 8 angelenkt sein, an der weitere Kraftübertragungseinrichtungen 10 gekoppelt sind, die weitere Fingerprothesen 3, 4, 5 aktiv anlenken.

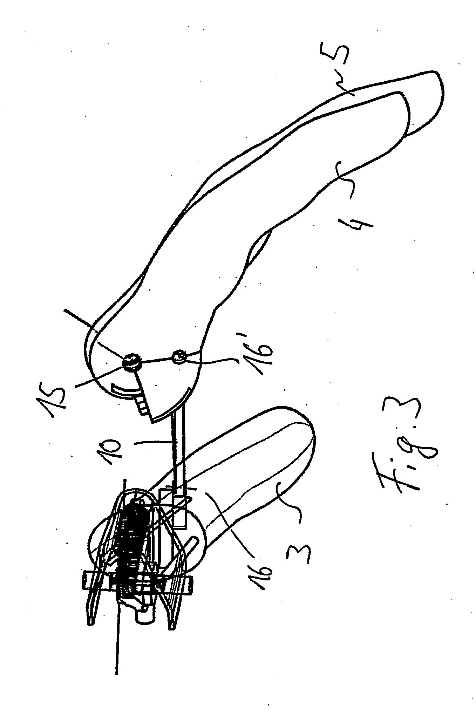

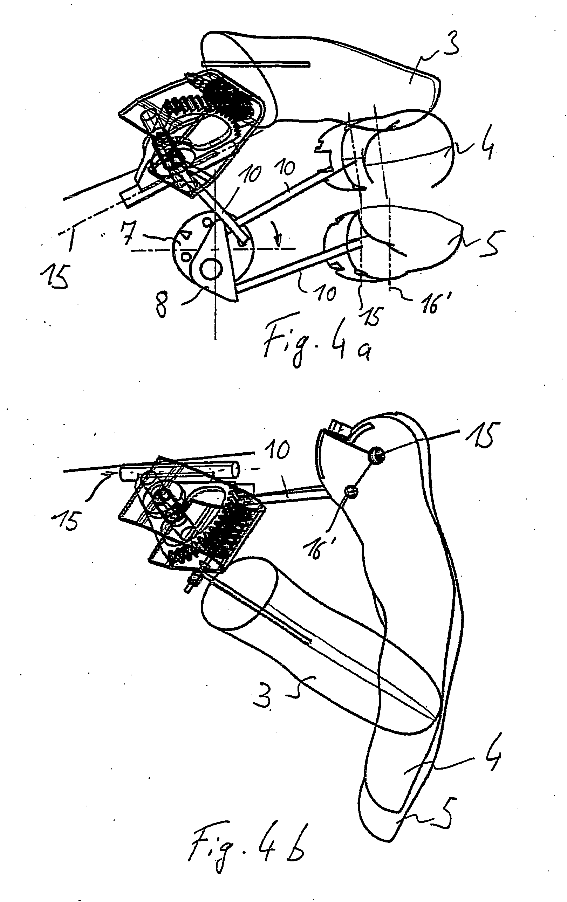

In der Figur 3 ist in einer Seitenansicht der Figur 2 die Handprothese in einer Ruhestellung gezeigt, in der der Daumen 3, der Zeigefinger 4 und der Mittelfinger 5 in einer leicht geöffneten, der natürlichen Handhaltung angenäherten Ruhestellung dargestellt sind. Dieser Figur ist zu entnehmen, dass die Kraftübertragungseinrichtungen 10 an Lagerstellen 16' an den Fingerprothesen 4, 5 angelenkt sind, die zu den Drehachsen 15 der Fingerprothesen 4, 5 beabstandet sind. Durch eine Verlagerung der Drehachse 16 auf dem Kopplungselement 7 wird eine Beugung der Fingerprothesen 4, 5 aufgrund der übertragenen Zugkräfte bewirkt. Wird nun, ausgehend von der in den Figuren 2 und 3 gezeigten Ruhestellung, die Drehscheibe 7, wie in der Figur 4a gezeigt, im Uhrzeigersinn gedreht, bewegen sich zunächst die Zeige- und Mittelfingerprothesen 4, 5 in Richtung Handinnenfläche, während die Daumenprothese 3 erst danach in Richtung Handinnenfläche verlagert wird, da die Kraftübertragungseinrichtung 10, die der Daumenprothese 3 zugeordnet ist, zunächst den Totpunkt, also den kürzesten Abstand zwischen der antriebsseitigen Drehachse 16 und der Schwenkachse 15 durchlaufen muss. Durch die spezielle Anordnung der Kraftübertragungseinrichtungen 10 der Zeige- und Mittelfingerprothesen 4, 5 werden diese schneller bzw. über einen weiteren Winkelbereich palmar verlagert, so dass die Daumenprothese 3 an der radialen Seite der Zeigefingerprothese 4 anliegt. Dadurch wird der dargestellte Lateralgriff ermöglicht.

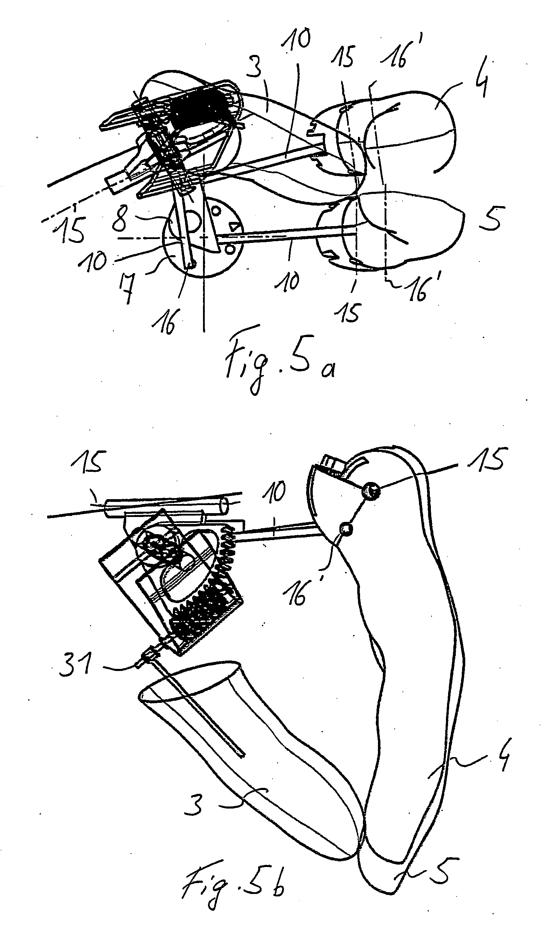

In der Figur 5 ist die Stellung der Fingerprothesen 3, 4, 5 bei einer Drehrichtung entgegen dem Uhrzeigersinn gezeigt.

Dort wird zunächst die Daumenprothese 3 palmar und ulnar um die Schwenkachse 15 bewegt, während die Fingerprothesen 4, 5 erst ihren Totpunkt durchlaufen müssen bzw. so an der Drehscheibe 7 angelenkt sind, dass bei einem entsprechenden Drehwinkel nur eine geringere Winkelverlagerung realisiert wird. So wird die Daumenprothese 3 zunächst nach innen geführt und die Spitzen der Fingerprothesen 3, 4, 5 liegen in ihren Endstellungen aneinander an, so dass ein sogenannter "Spitzgriff" realisiert wird.

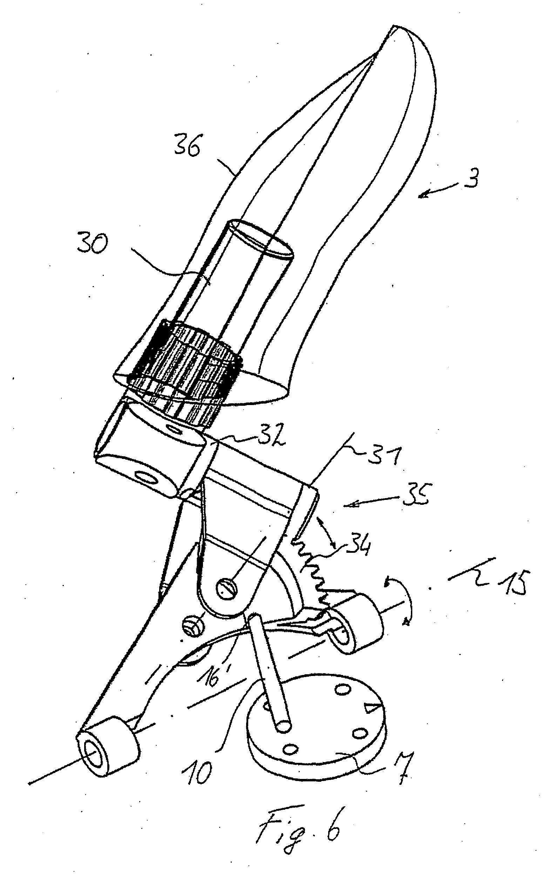

Um noch weitere Griffmöglichkeiten bereitstellen zu können, kann in der Daumenprothese 3 ein zusätzlicher Antrieb vorgesehen sein, wie er in der Figur 6 gezeigt ist. In der Figur 6 ist weiterhin zu erkennen, dass die Daumenprothese 3 neben der ersten Schwenkachse 15 eine zweite Schwenkachse 31 aufweist, um die zumindest das distale Ende der Daumenprothese 3 verschwenkbar gelagert ist. Über einen zweiten Antrieb 30 und ein Schrägschraubgetriebe 32 oder ein mehrgängiges Schneckengetriebe wird eine Abtriebsschnecke 33 bewegt, die mit einem Zahnradsegment 34 kämmt und so eine Verlagerung der Fingerprothese 3 mit dem Antrieb 30 und dem Getriebe 32 um die Schwenkachse 31 bewirkt. Werden beide Antriebe 6, 30 gleichzeitig aktiviert, wird entsprechend der Verlagerungsgeschwindigkeiten eine Kombinationsbewegung der Daumenprothese 3 palmar und ulnar ausgeführt, was einer natürlichen Daumenbeweglichkeit entspricht.

In dieser Figur ist im Detail der Funktion der Daumenprothese 3 gezeigt, mit einem Formkörper 36, der der Kontur eines natürlichen Daumens nachgebildet ist. Innerhalb des Formkörpers 36, der als Hohlkörper ausgebildet ist, ist ein Freiraum, in dem der zweite Antrieb 30 angeordnet und befestigt ist. Der Formkörper 36 ist somit an den Antrieb 30 gekoppelt, beispielsweise angeklebt, festgeklemmt oder formschlüssig verbunden. Der Antrieb 30 ist über ein Winkelgetriebe in Gestalt eines Schrägschraubgetriebes 32 und der in der Figur 2 beschriebenen Schnecke 33 mit dem Zahnradsegment 34 gekoppelt.

Bei Aktivierung des Antriebes 30 wird die Schnecke 33 in die eine oder andere Richtung gedreht. Aufgrund der schwenkbaren Lagerung um die Drehachse 31 an dem Zahnradsegment 34 ist eine Bewegung um die Schwenkachse 31 in Richtung des Doppelpfeiles möglich. Es kann dabei eine radiale oder ulnare Bewegung ausgeführt werden. Das Zahnradsegment 34 selbst ist schwenkbar um die erste Schwenkachse 15 gelagert und kann durch eine Drehung der Drehscheibe 7 und die dadurch bewirkte Verlagerung des Kraftübertragungselementes 10 palmar oder dorsal verschwenkt werden. Diese Verschwenkbewegung ist ebenfalls durch den Doppelpfeil um die Schwenkachse 15 angedeutet.

Der zweite Antrieb 30 ist ebenfalls ein Elektromotor und befindet sich bevorzugt in der Längsachse zwischen dem Sattel- und dem Interphalangialgelenk. Aufgrund der kleinen Bauart und dem ggf. notwendigen, hohen Antriebsmoment ist der Antrieb 30 als Schnelläufer ausgebildet, wobei das Übersetzungsgetriebe 32 als Schrägschraubgetriebe ausgebildet sein kann und eine Umlenkung der Abtriebsachse relativ zu der Längsachse des zweiten Antriebs 30 in einem Winkelbereich von 45° bis 135° erzeugt. Aufgrund dieser Abwinklung der Abtriebsachse ist es möglich, dass die Schnecke 33, die das Zahnradsegment 34 kämmt, eine entsprechende Daumenbewegung bewirkt.

Der in dem Handchassis 2 angeordnete erste Antrieb 6 ist bevorzugt ein langsam laufender Flachläufermotor mit einem hohen Drehmoment, der mit einem hochuntersetzten Getriebe gekoppelt ist, um eine entsprechend langsame und kraftvolle Greifbewegung ausführen zu können. Die Steuerungssignale können entweder von einer Fernsteuerung oder myoelektrische Signale und ein Steuergerät generiert werden. Über diesen ersten Antrieb 6 und die Drehscheibe 7 ist das Zahnradsegment 34 mitsamt der Schnecke 33 sowie dem Getriebe 32 und dem Antrieb 30, abgedeckt durch den Formkörper 36, verlagerbar.

## Patentansprüche

1. Handprothese mit einem Chassis, an dem mehrere Fingerprothesen gelenkig oder elastisch gelagert sind, die über einen Antrieb um zumindest eine Schwenkachse relativ zu dem Chassis und aufeinander zu bewegbar sind, **dadurch gekennzeichnet, dass** die Kraftübertragungseinrichtungen (10) an einem gemeinsamen Antrieb (6) über ein Kopplungselement (7) dergestalt mit den Fingerprothesen (3, 4, 5) gekoppelt sind, dass ausgehend von einer Ruhestellung in Abhängigkeit von der Drehrichtung des Antriebes (6) zumindest zwei Fingerprothesen (3, 4, 5) unterschiedliche Verstellwinkel in die selbe Richtung relativ zu dem Chassis (2) durchlaufen, wobei die Kraftübertragungseinrichtungen (10) dergestalt mit dem Kopplungselement (7) gekoppelt sind, dass ihre antriebsseitigen Lagerungen unterschiedliche Totpunktlagen aufweisen.

2. Handprothese nach Anspruch 1, **dadurch gekennzeichnet, dass** die Kraftübertragungseinrichtungen (10) drehbar an dem verschwenkbaren Kopplungselement (7) gelagert sind.

3. Handprothese nach Anspruch 1 oder 2, **dadurch gekennzeichnet, dass** das Kopplungselement (7) als Drehscheibe ausgebildet ist.

4. Handprothese nach Anspruch 2 oder 3, **dadurch gekennzeichnet, dass** die Drehachse des Kopplungselementes (7) im Wesentlichen orthogonal zu der palmaren Oberfläche des Chassis (2) ausgerichtet ist.

5. Handprothese nach einem der voranstehenden Ansprüche, **dadurch gekennzeichnet, dass** die Abtriebsachse des Antriebes (6) im Wesentlichen orthogonal zu der palmaren Oberfläche des Chassis (2) ausgerichtet ist.

6. Handprothese nach einem der voranstehenden Ansprüche, **dadurch gekennzeichnet, dass** der Antrieb (6) als Flachläufermotor ausgebildet ist.

7. Handprothese nach einem der voranstehenden Ansprüche, **dadurch gekennzeichnet, dass** der Antrieb (6) mit einem Zykloiden-Getriebe oder einem Harmonic-Drive-Getriebe gekoppelt ist, an dem das Kopplungselement (7) angeordnet ist.

8. Handprothese nach einem der voranstehenden Ansprüche, **dadurch gekennzeichnet, dass** die Kraftübertragungseinrichtung (10) zugstarr und drucknachgiebig oder biegeelastisch ausgebildet ist.

9. Handprothese nach einem der voranstehenden Ansprüche, **dadurch gekennzeichnet, dass** die Kraftübertragungskomponente (10) eine Seil-, Litzen- oder Faserkomponente aufweist.

10. Handprothese nach Anspruch 9, **dadurch gekennzeichnet, dass** die Seil-, Litzen- oder Faserkomponente als geschlossene Schlaufe ausgebildet ist.

11. Handprothese nach einem der voranstehenden Ansprüche, **dadurch gekennzeichnet, dass** die Kraftübertragungseinrichtung (10) eine Elastomerkomponente aufweist.

12. Handprothese nach Anspruch 9 und 11, **dadurch gekennzeichnet, dass** die Elastomerkomponente die Seil-, Litzen- oder Faserkomponente zumindest teilweise umschließt.

13. Handprothese nach einem der voranstehenden Ansprüche, **dadurch gekennzeichnet, dass** die Kraftübertragungseinrichtungen (10) Lagerbuchsen zur Aufnahme von Achsen (15, 16) aufweisen, die dem Chassis (2) und den Fingerprothesen (3, 4, 5) zugeordnet sind.

14. Handprothese nach einem der voranstehenden Ansprüche, **dadurch gekennzeichnet, dass** die Kraftübertragungseinrichtung (10) federelastisch ausgebildet ist.

15. Handprothese nach Anspruch 14, **dadurch gekennzeichnet, dass** die Federrate der Kraftübertragungseinrichtung (10) so bemessen ist, dass bei einer Beaufschlagung der Kraftübertragungseinrichtung (10) mit einer Druckkraft eine Rückstellung der Fingerprothese (3, 4, 5) in eine Ausgangsstellung bewirkt wird.

## Claims

1. A hand prosthesis comprising a chassis, to which a number of finger prostheses are articulated or elastically coupled, said finger prostheses being movable relative to the chassis and toward one another about at least one swiveling axis by means of a drive, **characterized in that** the force transmission units (10) on a common drive (6) by means of a coupling element (7) are coupled to the finger prostheses (3, 4, 5) in such a way that, starting from a rest position depending on the direction of rotation, at least two finger prostheses (3, 4, 5) go through different adjusting angles in the same direction relative to the chassis (2) wherein the force transmission units (10) are coupled to the coupling element (7) in such a way that their mountings on the drive side have different dead center positions.

2. The hand prosthesis as claimed in claim 1, **characterized in that** the force transmission unit (10) are rotatably mounted on a swiveling coupling element (7).

3. The hand prosthesis as claimed in claim 1 or 2, **characterized in that** the coupling element (7) is formed as a rotary disk.

4. The hand prosthesis as claimed in claim 2 or 3, **characterized in that** the axis of rotation of the coupling element (7) is aligned substantially orthogonal to the palmar surface of the chassis (2).

5. The hand prosthesis as claimed in one of the preceding claims, **characterized in that** the output axis of the drive (6) is aligned substantially orthogonal to the palmar surface of the chassis (2).

6. The hand prosthesis as claimed in one of the preceding claims, **characterized in that** drive (6) is formed as a pancake motor.

7. The hand prosthesis as claimed in one of the preceding claims, **characterized in that** the drive (6) is coupled to a cycloidal gear mechanism or aharmonic-drive gear mechanism on which the coupling element (7) is located.

8. The hand prosthesis as claimed in one of the preceding claims, **characterized in that** the force transmission unit (10) is formed so as to be rigid under tension and yielding under pressure or elastic under bending.

9. The hand prosthesis as claimed in one of the preceding claims, **characterized in that** the force transmission unit (10) has a cable, stranded-wire or fiber component.

10. The hand prosthesis as claimed in claim 9 **characterized in that** the cable, stranded-wire or fiber component is formed as a closed loop.

11. The hand prosthesis as claimed in one of the preceding claims, **characterized in that** the force transmission unit (10) has an elastomer component.

12. The hand prosthesis as claimed in claims 9 and 11, **characterized in that** the elastomer component at least partially encloses the cable, stranded-wire or fiber component.

13. The hand prosthesis as claimed in one of the preceding claims, **characterized in that** the force transmission units (10) have bearing bushes for receiving axial spindles (15, 16) that are assigned to the chassis (2) and the finger prostheses (3,4,5).

14. The hand prosthesis as claimed in one of the preceding claims, **characterized in that** the force transmission unit (10) is formed in a resiliently elastic manner.

15. The hand prosthesis as claimed in claim 15, **characterized in that** the spring rate of the force transmission unit (10) is set such that, when the force transmission unit (10) is subjected to the force of a pressure, a return of the finger prosthesis (3, 4, 5) into a starting position is brought about.

## Revendications

1. Prothèse de main comprenant un châssis sur lequel sont montées de façon articulée ou élastique plusieurs prothèses de doigt, qui sont mobiles relativement au châssis et les unes vers les autres, autour d'au moins un axe de pivotement et par l'intermédiaire d'un entraînement, **caractérisée en ce que** les dispositifs de transmission de force (10) sont couplés à un entraînement commun (6) par l'intermédiaire d'un élément de couplage (7), et sont couplés avec les prothèses de doigt (3, 4, 5) de sorte qu'au moins deux prothèses de doigt (3, 4, 5) se déplacent sur des angles différents dans le même sens relativement au châssis (2), en fonction du sens de rotation de l'entraînement (6), à partir d'une position de repos, les dispositifs de transmission de force (10) étant couplés à l'élément de couplage (7) de façon que leurs montages côté entraînement présentent différentes positions de point mort

2. Prothèse de main selon la revendication 1, **caractérisée en ce que** les dispositifs de transmission de force (10) sont montés rotatifs sur l'élément de couplage (7) pivotable.

3. Prothèse de main selon la revendication 1 ou 2, **caractérisée en ce que** l'élément de couplage (7) est formé par une plaque tournante.

4. Prothèse de main selon la revendication 2 ou 3, **caractérisée en ce que** l'axe de rotation de l'élément de couplage (7) est orienté sensiblement orthogonalement à la surface palmaire du châssis (2).

5. Prothèse de main selon l'une quelconque des revendications précédentes, **caractérisée en ce que** l'axe de sortie de l'entraînement (6) est orienté sensiblement orthogonalement à la surface palmaire du châssis (2).

6. Prothèse de main selon l'une quelconque des revendications précédentes, **caractérisée en ce que** l'entraînement (6) est formé par un moteur à rotor plat.

7. Prothèse de main selon l'une quelconque des revendications précédentes, **caractérisée en ce que** l'entraînement (6) est couplé à une transmission cycloïdale ou une transmission à onde de déformation, sur laquelle est agencé l'élément de couplage (7).

8. Prothèse de main selon l'une quelconque des revendications précédentes, **caractérisée en ce que** le dispositif de transmission de force (10) est formé résistant à la traction et compressible, ou élastiquement flexible.

9. Prothèse de main selon l'une quelconque des revendications précédentes, **caractérisée en ce que** l'élément de transmission de force (10) présente un élément câble, cordon ou fibre.

10. Prothèse de main selon la revendication 9, **caractérisée en ce que** l'élément câble, cordon ou fibre est formé par une boucle fermée.

11. Prothèse de main selon l'une quelconque des revendications précédentes, **caractérisée en ce que** le dispositif de transmission de force (10) présente un élément en élastomère.

12. Prothèse de main selon les revendications 9 et 11, **caractérisé en ce que** l'élément en élastomère entoure au moins partiellement l'élément câble, cordon ou fibre.

13. Prothèse de main selon l'une quelconque des revendications précédentes, **caractérisée en ce que** les dispositifs de transmission de force (10) présentent des coussinets de paliers pour recevoir des axes (15, 16) associés au châssis (2) et aux prothèses de doigt (3, 4, 5).

14. Prothèse de main selon l'une quelconque des revendications précédentes, **caractérisée en ce que** le dispositif de transmission de force (10) est formé à retour élastique.

15. Prothèse de main selon la revendication 14, **caractérisée en ce que** le taux d'élasticité du dispositif de transmission de force (10) est déterminé de telle façon qu'il se produise un rappel des prothèses de doigt (3, 4, 5) dans une position initiale, lors de l'application d'une force de pression sur le dispositif de transmission de force (10).
